# EUROPÄISCHE PATENTANMELDUNG

(11) **EP 0 592 709 A1**
(43) Veröffentlichungstag der Anmeldung: **20.04.1994**
(21) Anmeldenummer: 92117524.6
(22) Anmeldetag: 14.10.1992
(51) Int. Cl.: A61H 39/00, A61N 1/18, A44C 7/00

(54) **Ohrschmuck**

(71) Anmelder: Hieber, Fritz E.W. Dr. med., D-76530 Baden-Baden (DE)
(72) Erfinder: Hieber, Fritz E.W., Dr. med., D-76530 Baden-Baden (DE); Trappenberg, Hans, W-7500 Karlsruhe 21 (DE)
(74) Vertreter: Trappenberg, Hans

(57) **Zusammenfassung**

"Therapeutische Ohrringe", die mit zweierlei Materialien versehen sind, die zusammen mit dem insbesondere in der Ohrmuschelgrube befindlichen Elektrolyten ein galvanisches Element bilden, werden bisher aus Edelmetallen oder sonstigen wertvollen Metallverbindungen gefertigt. Eine preiswerte Herstellung ergibt sich nach der Erfindung durch die Verwendung von Kunststoff für den Schmuckstückkörper, der mit das gewünschte Spannungspotential aufweisenden Kontaktflächen versehen ist.

## Beschreibung

Die Erfindung betrifft Ohrschmuck zur Stimulation von an der Ohrmuschel vorhandenen Akupunkturpunkten beziehungsweise -Linien, gebildet durch ein etwa offenkreisringförmig gebogenes Schmuckstück beliebiger Form, dessen eine Stirnseite zum Einhängen in die Ohrmuschelgrube bestimmt ist, während sich das andere Ende an der Ohrmuschelhinterwand abstützt, oder das Schmuckstück vorderseitige Schauflächen aufweist und die Ohrmuschel hintergreift, wobei mit der Ohrmuschel in Kontakt kommende Schmuckstückflächen aus Materialien, vorzugsweise aus Metallen, gebildet sind, die entsprechend der elektrochemischen Spannungsreihe ein gegenseitiges Potential aufweisen.

Bei derartigem Ohrschmuck entsteht durch die an den Kontaktflächen des Ohrschmucks vorhandenen unterschiedlichen Materialien, zusammen mit dem Säuremantel der Haut ein galvanisches Element, was geeignet ist durch den steten Stromfluß Akupunkturwirkungen hervorzurufen. Hierbei liegt der eine Endpunkt des Ohrschmucks in der Ohrmuschelgrube fest, während der zweite Endpunkt frei an der Hinterwand der Ohrmuschel wählbar ist. Dadurch können auch Akupunkturpunkte beziehungsweise -Linien erreicht werden, die beispielsweise durch übliche Kreolen nicht belegt werden können.

Entsprechend dem Verwendungszweck wurde bisher derartiger Ohrschmuck ausschließlich aus Edelmetall gefertigt, um sowohl einer Reizung der belegten Hautpartien, wie insbesondere auch der Auslösung von Allergien vorzubeugen. Dadurch jedoch wurde derartiger Ohrschmuck schon vom Material her sehr teuer. Hinzu kam noch die aufwendige Herstellung, die insbesonders durch das Einfügen des andersartigen Materials an der einen Stirnfläche bedingt war. Um dem auszuweichen wurde auch schon vorgeschlagen, den Schmuckkörper aus Gußbronze herzustellen mit einem am einen Ende eingegossenen Endstück aus Eisen.

Auch diese verhältnismäßig preiswerte Herstellung erscheint noch zu teuer, um diesen "therapeutischen Ohrring" größeren Kreisen zugänglich zu machen. Es ist daher Aufgabe der Erfindung, eine Möglichkeit der Herstellung anzugeben, die so preiswert ist, daß ein solches Schmuckstück etwa in der Preislage der sonstigen Schmuckstücke liegt, die nicht diese therapeutische Wirkung zeigen. Erreicht wird dies nach der Erfindung dadurch, daß der Ohrschmuckkörper aus Kunststoff geformt ist, an dem die ein gegenseitiges Potential aufweisenden Materialien an den Kontaktstellen aufgebracht sind.

Nicht mehr also wie bisher wird das Schmuckstück aus einem der Materialien beziehungsweise einem der Metalle, insbesondere aus Gold gefertigt, sondern aus preiswertem Kunststoff, der danach so behandelt wird, daß diese gewünschte therapeutische Wirkung durch das Aufbringen der verschiedenen Materialien gewährleistet ist. So kann der Kunststoff-Ohrschmuckkörper galvanisch leitend beschichtet sein, wobei die Beschichtung eine sehr geringe Dicke aufweisen kann, da es sich bei den hier fließenden Strömen auch nur um äußerst geringe Stromstärken handelt, wobei die Beschichtung bereits aus einem solchen Material gebildet werden kann, das das gewünschte Potential zu einem weiteren Material aufweist. Die Kontaktstellen können hierbei die Stirnflächen des offen-kreisförmig gebogenen Schmuckstücks sein, die galvanisch durch die Beschichtung verbunden sind. Das Material an den Kontaktstellen kann hierbei auf die Beschichtung aufgedampft sein oder es kann auch in Form einer Hülse über die Endteile des Schmuckstücks übergeschoben werden. Zusätzlich zu diesen stirnseitigen Kontaktstellen können auch weitere Kontaktstellen auf der Beschichtung angebracht werden, indem Metallauflagen auf die Beschichtung an den den Akupunktur-Punkten entsprechenden Stellen aufgebracht werden. Eine sehr preiswerte Herstellmöglichkeit ergibt sich auch ohne ganzflächige Beschichtung des Schmuckstücks dadurch, daß eine aus Metall bestehende Hülse auf die Endteile des Schmuckstücks oder auch auf nur ein Endteil aufgesteckt wird, das aus zwei galvanisch miteinander verbundenen Metallen gebildet ist, zwischen denen an der Auflagestelle an die Ohrmuschel eine Nut vorgesehen ist, so daß sich an dieser Stelle sowohl zusammen mit dem beispielsweise in der Ohrmuschelgrube befindlichen Elektrolyten ein galvanisches Element bildet, wie auch die Wirkungen dieses galvanischen Elementes sich auf dieses Ohrmuschel-Areal auswirken. Vorteilhafterweise werden in allen diesen Fällen als Spannungspotential aufweisende Materialien einerseits Gold, andererseits Eisen eingesetzt.

Auf der Zeichnung sind Ausführungsbeispiele schematisch dargestellt, und zwar zeigen:

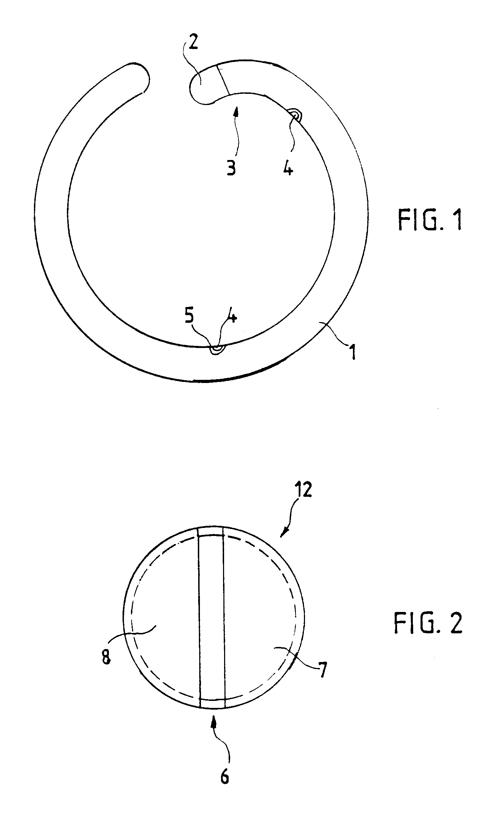

- Fig. 1: eine erste Ausführungsform eines solchen Schmuckstücks
und
- Fig. 2: die Draufsicht auf eine Stirnkappe.

Ein offen-kreisringförmig gebogenes, aus Kunststoff bestehendes Schmuckstück (1) ist auf der gesamten Oberfläche mit einem Material beschichtet, das zu einem weiteren Material, das als Hülse (2) auf ein Endteil des Schmuckstücks aufgesetzt ist, ein Potential nach der elektrochemischen Spannungsreihe aufweist. Das Endteil (3) mit der Hülse (2) ist leicht nach innen gebogen, um einen sicheren Sitz in der Ohrmuschelgrube zu erreichen. Zu dem Beschichtungsmaterial des Schmuckstücks ein Spannungspotential aufweisendes Material ist außerdem noch an weiteren Arealen des Schmuckstücks (1), die mit dem Gewebe des Ohrläppchens in Verbindung kommen, angebracht. Dieses Material (4) ist durch ein isolierendes Material (5) von dem Beschichtungsmaterial getrennt, so daß sich dort auch wiederum, zusammen mit dem auf der Haut befindlichen Elektrolyten, ein galvanisches Element bildet.

Bei einer weiteren Ausführung benötigt das Schmuckstück keine Beschichtung, jedoch ist eine Hülse (12) so ausgebildet, daß zwei Metalle, die gegenseitig ein Potential nach der elektrochemischen Spannungsreihe aufweisen, nebeneinander, durch eine Nut (6) getrennt, angeordnet sind. Dadurch entsteht auch hier wiederum, zusammen mit dem in der Ohrmuschelgrube befindlichen Elektrolyt, ein galvanisches Element, dessen Strom sich an dieser Stelle auf das Gewebe der Ohrmuschel auswirkt.

## Patentansprüche

1. Ohrschmuck zur Stimulation von an der Ohrmuschel vorhandenen Akupunkturpunkten beziehungsweise -Linien, gebildet durch ein etwa offen-kreisringförmig gebogenes Schmuckstück beliebiger Form, dessen eine Stirnseite zum Einhängen in die Ohrmuschelgrube bestimmt ist, während sich das andere Ende an der Ohrmuschelhinterwand abstützt, oder das Schmuckstück vorderseitige Schauflächen aufweist und die Ohrmuschel hintergreift, wobei mit der Ohrmuschel in Kontakt kommende Schmuckstückflächen aus Materialien, vorzugsweise aus Metallen, gebildet sind, die entsprechend der elektrochemischen Spannungsreihe ein gegenseitiges Potential aufweisen,
dadurch gekennzeichnet,
daß der Ohrschmuckkörper (1) aus Kunststoff geformt ist, an dem die ein gegenseitiges Potential aufweisende Materialien (4, 7, 8) an den Kontaktstellen aufgebracht sind.

2. Ohrschmuck nach Anspruch 1,
dadurch gekennzeichnet,
daS der Ohrschmuckkörper galvanisch leitend beschichtet ist.

3. Ohrschmuck nach Anspruch 2,
dadurch gekennzeichnet,
daß die Beschichtung aus einem Material ist, das zusammen mit dem weiteren Material (4) an der Kontaktstelle das gegenseitige Spannungspotential aufweist.

4. Ohrschmuck nach einem oder mehreren
der vorhergehenden Ansprüche,
dadurch gekennzeichnet,
daS die Kontaktstellen die Stirnflächen des offenkreisringförmig gebogenen Schmuckstücks sind und daß die Kontaktstellen über die Beschichtung galvanisch miteinander verbunden sind.

5. Ohrschmuck nach Anspruch 4,
dadurch gekennzeichnet,
daS die Kontaktstellen auf die Endteile des offenkreisringförmig gebogenen Schmuckstücks aufgesetzte Hülsen sind.

6. Ohrschmuck nach einem oder mehreren
der vorhergehenden Ansprüche,
dadurch gekennzeichnet,
daS die Kontaktstellen auf die Beschichtung aufgesetzte und mit ihr galvanisch verbundene Metallauflagen (4) sind, die zu dem Beschichtungsmaterial ein elektrochemisches Spannungspotential aufweisen.

7. Ohrschmuck nach Anspruch 1,
dadurch gekennzeichnet,
daß die ein gegenseitiges Spannungspotential aufweisenden Materialien an einer ein- oder beidseitig auf die Endteile (3) des Schmuckstücks aufsteckbaren Hülse (12) angeordnet sind.

8. Ohrschmuck nach Anspruch 7,
dadurch gekennzeichnet,
daS die Hülse (12) aus einem Metall (7) gefertigt ist, auf das galvanisch mit ihm verbunden ein weiteres, zu dem Grundmetall (7) ein elektrochemisches Spannungspotential aufweisendes Metallstück (8) so aufgesetzt ist, daß sich die beiden Metalle (7, 8) an der Kontaktstelle nicht berühren.

9. Ohrschmuck nach einem oder mehreren
der vorhergehenden Ansprüche,
dadurch gekennzeichnet,
daß die ein gegenseitiges elektrochemisches Spannungspotential aufweisenden Materialien (4, 7, 8) Gold und Eisen sind.
